Europäisches Patentamt

European Patent Office

Office européen des brevets

(11) Publication number: **0 314 278**

**A1**

# EUROPEAN PATENT APPLICATION

(21) Application number: **88307915.4**

(22) Date of filing: **26.08.88**

(51) Int. Cl.⁴: **A61M 5/32**

(30) Priority: **29.09.87 JP 245687/87**
**20.10.87 JP 265664/87**

(43) Date of publication of application:
**03.05.89 Bulletin 89/18**

(84) Designated Contracting States:
**CH DE FR GB IT LI**

(71) Applicant: **Steinberg, Danny David Charles**
**Sunrise Oyama Mansion 401 54-15 Oyama**
**Kanai-Cho**
**Itabashi-ku Tokyo 173(JP)**

(72) Inventor: **Steinberg, Danny David Charles**
**Sunrise Oyama Mansion 401 54-15 Oyama**
**Kanai-Cho**
**Itabashi-ku Tokyo 173(JP)**

(74) Representative: **Hayward, Denis Edward Peter**
**et al**
**Lloyd Wise, Tregear & Co. Norman House**
**105-109 Strand**
**London WC2R 0AE(GB)**

(54) **Needle Shields.**

(57) One of the main features of this invention is the shape of the cap which is to cover the needle (5). The other main feature is that of a disc-like object (25) into which a receptacle (9) for a needle (5) is inserted. The disc (25) is constructed of a material that is impervious to the thrust of the needle (5).

FIG. 8

## NEEDLE SHIELDS

This invention protects a user's hands and fingers from being pierced or pricked by a needle when (1) the user is replacing the cap of a used needle, or (2) the user is holding the receptacle into which a used needle is to be inserted. A person who pierces or pricks his or her own skin is in danger of receiving harmful viruses, bacteria and other organisms, if the the needle is carrying such life forms. AIDS, Hepatitis B and other deadly diseases may be contracted in this way. Accidents are more likely to occur when the user is busy or fatigued.

Prior caps do not provide sufficient protection for a person when that person replaces the cap on a used needle. Protection is not provided because the diameter of the cap is of a minimal size so that it will fit closely around the needle. The result is that many users inadvertently miss capping the needle and consequently pierce or prick their own fingers or hand. Replacing the caps on used needles before discarding is a common safety practice, particularly for medical personnel. Nurses, for example, replace the caps on syringes after drawing a blood sample from patients. They also replace the caps on the needles which are connected to intravenous feeding devices after patients have received feeding.

Protection is similarly needed when a person has to insert a used needle into a receptacle such as a test tube. A user may inadvertently miss the entrance of the receptacle with result that the needle pierces or pricks his or her own skin. Accidents of this sort may occur when nurses, for example, draw blood with a needle and then must transfer the blood to a receptacle. The nurse may miss the top of the test tube when making a thrust with the needle of the syringe and then strike his or her own fingers or hand.

Fig. 1 shows a typical situation where Needle 5 is being inserted into Cap 1 via Mouth 7.

Fig. 2 shows a typical situation where blood that has been drawn from a patient is being transferred into a test tube receptacle. Usually Test Tube 9 has a Stopper 11. The Hand 3 holds Test Tube 9 stable so that the needle can be inserted through Stopper 11. After the blood has been transferred, the needle is withdrawn.

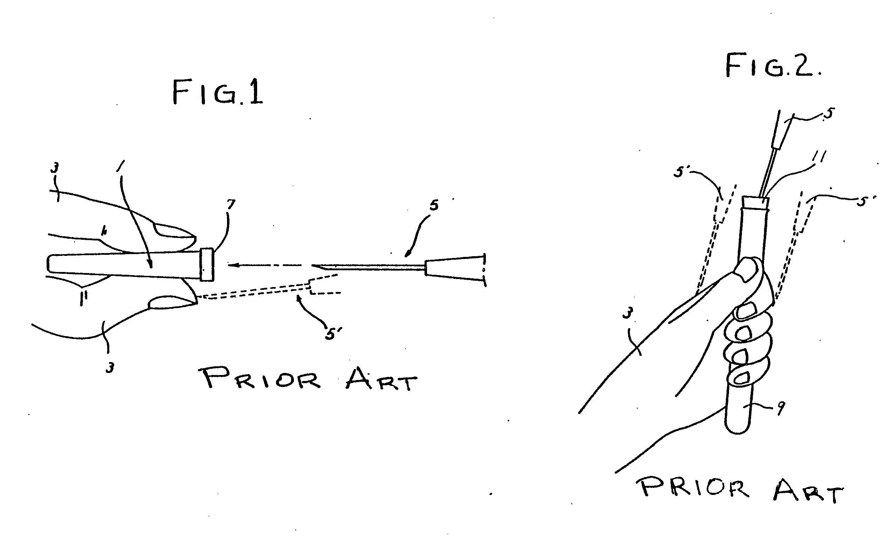

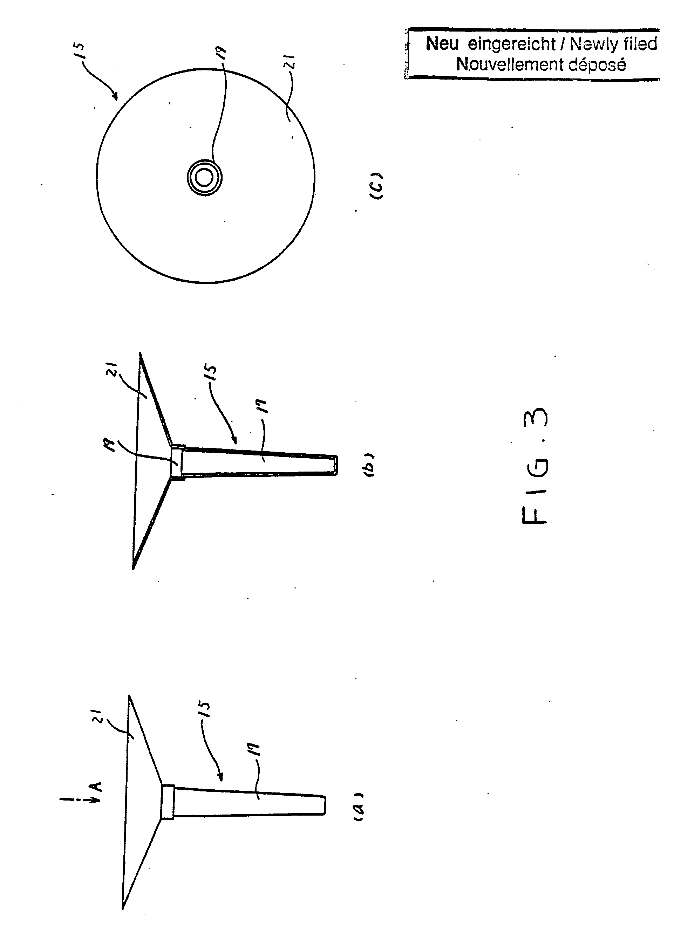

Concerning Fig. 1, there is a danger that a user may pierce or prick his or her finger or hand, as shown by Dotted Line 5'. In Fig. 2, the Dotted Lines 5' show a similar situation.

This invention takes into account the aforementioned situations by giving protection to the parts of the body, mainly fingers and hands, which hold a cap or receptacle. The effect of the invention is to prevent the fingers or hands from being pierced or pricked by a needle bearing contaminated blood.

One of the main features of this invention is the shape of the cap which is to cover the needle. The other main feature is that of a disc-like object into which a receptacle for a needle is inserted. The disc is constructed of a material that is impervious to the thrust of the needle.

Fig. 1 shows a situation where a used needle is covered by a typical cap, Cap 1.

Fig. 2 shows a typical situation where blood is transferred into Test Tube 9.

Fig. 3 shows one invention, Needle Shield 15, where (a) presents a side view, (b) presents a cross-sectional view, and (c) presents a top view.

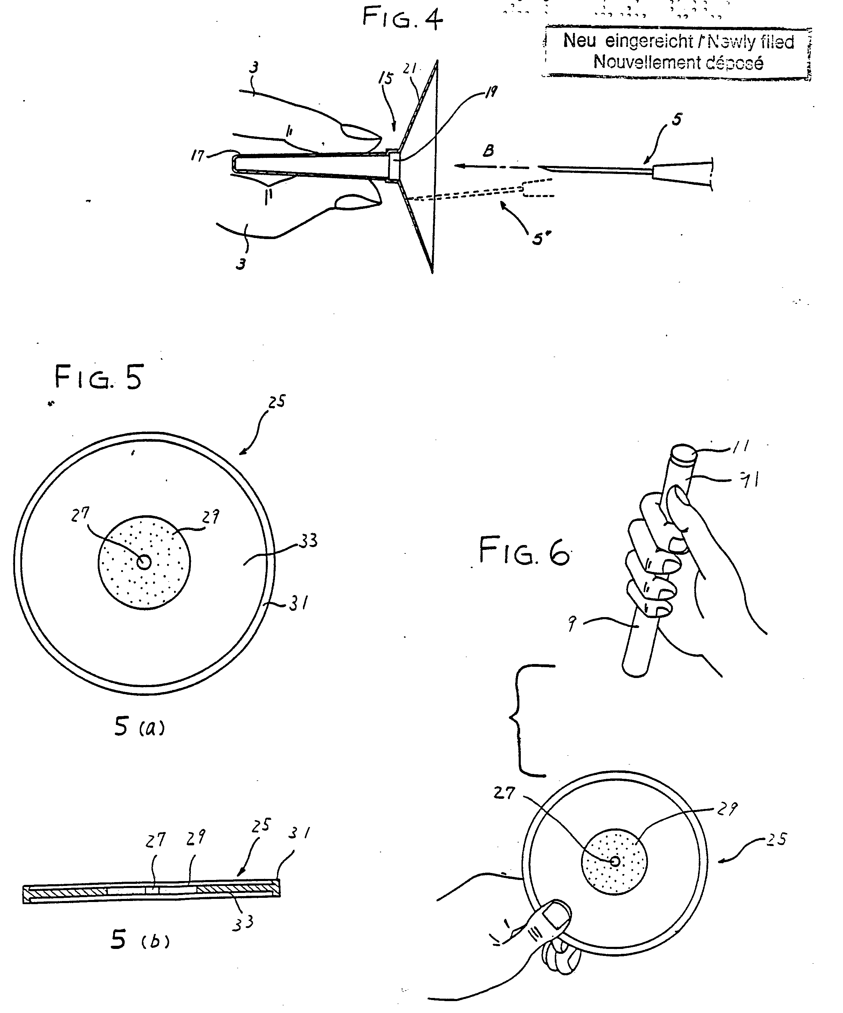

Fig. 4 demonstrates how Needle Shield 15 is protectively used. If Needle 5 misses Mouth 19, it will strike impervious Shield 21 of the cap.

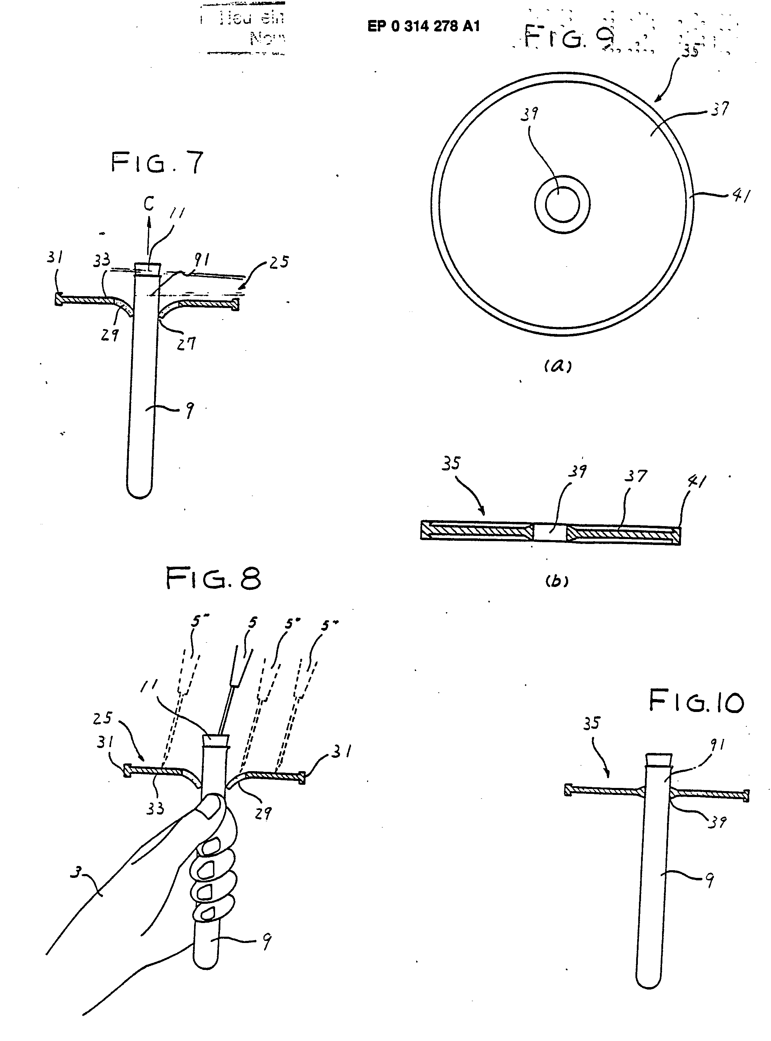

Fig. 5 shows the second invention, Holder Shield 25, where (a) presents a top view, and (b) presents a cross-sectional view.

Fig. 6 shows how Test Tube 9 is to be inserted through the Holding Aperture 27 of Holder Shield 25.

Fig. 7 shows Test Tube 9 secured in Holding Aperture 27 after having been inserted.

Fig. 8 demonstrates how the Holder Shield is protectively used. If Needle 5 misses Stopper 11 of Test Tube 9, it will strike the impervious surfaces, 29 and 25.

Fig. 9 shows another example of the Holder Shield, 35, where it is all constructed of one kind of material. The top view is shown in (a) and the cross-sectional view is shown in (b).

Fig. 10 shows how Test Tube 9 is held in Holder Shield 35.

A detailed explanation of the Needle Shield invention follows.

Fig. 3 shows Needle Shield 15, where (a) presents a side view, (b) presents a cross-sectional view, and (c) presents a top view. As the drawing shows, Needle Shield 15 is comprised of Shield 21, which extends outward from Mouth 19 of Cap 17.

Cap 17 is shaped so as to envelop the needle that is inserted into it. The needle is inserted through the opening, Mouth 19. Shield 21 extends outward from Mouth 19 in a funnel-like shape.

Needle Shield 15 is made, typically, entirely of plastic.

Fig. 4 demonstrates how Needle Shield 15 is protectively used. The stem-like structure of Cap 17 is held by Fingers 3 while Needle 5 is inserted into Needle Shield 15. Needle 5 moves in Direction B through Mouth 19 and comes to rest inside of Cap 17. When this occurs, the handling of Needle 5 becomes safe. However, if when inserting Needle 5, the user misses Mouth 19, Needle 5 would strike Shield 21. This is shown in the figure by 5″. In this way, Fingers 3 holding Needle Shield 15 would be protected from Needle 5.

Needle Shield 15 can be easily constructed without drastically changing the original design of the typical needle cap. Just by extending Mouth 19 outwardly in a funnel-like fashion, the desired result can be obtained.

The above description of the Needle Shield is but one example of a number of embodiments which the Needle Shield may take. Four such embodiments are now presented.

(1) Shield 21 of the cap need not have a funnel-like shape. It could be flat, for example, and the aperture could be constant rather than increasing with length. Also, Shield 21 can be larger or smaller than the proportions shown in the Figures, depending on need.

(2) In the original embodiment described above, Shield 21 and Cap 17 were presented as one whole piece. However, Shield 21 and Cap 17 could be made in sections. These separate sections could then be assembled or connected in various ways such as by screwing them together.

(3) The Needle Shield 15 could be constructed of materials other than that of plastic, which was suggested above. Materials such as metals, e.g., aluminum, and rubber could be used.

(4) Cap 17 may have different shapes.

The Holder Shield will now be described in detail.

Fig. 5 shows the Holder Shield 25, where (a) presents a top view, and (b) presents a cross-sectional view. This Holder Shield 25 consists of a Receptacle Holding Area 29, and a Needle Protecting Area 33.

The Receptacle Holding Area 29 is circular and in its center there is a Holding Aperture 27 which is smaller than the diameter of Test Tube 9. The Receptacle Holder Area 29 is made of soft plastic or rubber, the thickness and strength of which is such that it cannot be penetrated by a thrust of Needle 5. Moreover, the surface of Receptacle Holder Area 29 is rough so that the Needle 5 will not slip or slide on it.

The Needle Protecting Area 33 is circular, and in the middle the Receptacle Holding Area 29 is secured. Around the perimeter of Needle Protecting Area 33 is a raised area, Ridge 31. This Ridge 31 extends both above and below the perimeter of Needle Protecting Area 33. The Needle Protecting Area 33 and the Ridge 31 are made of plastic, the thickness and strenth of which is such that it cannot be penetrated by a thrust of Needle 5. Moreover, the surface of Needle Protecting Area 33 is rough so that the Needle 5 will not slip or slide on it.

How Holder Shield 25 is used is now described.

First Test Tube 9 is inserted into the Holder Shield 25 as shown in Fig. 6. Insertion is performed by first passing the bottom of the Test Tube 9 through the Holding Aperture 27. Test Tube 9 is passed through the Holding Aperture 27 until it reaches the Upper Tube Section 91. This situation is shown in Fig. 7. As the drawing shows, because Receptacle Holding Area 29 is made of soft plastic or rubber, Holding Aperture 27 becomes enlarged when Test Tube 9 is inserted. In this way Test Tube 9 is tightly secured.

Fig. 8 shows how Holder Shield 25 is used. As shown in the drawing, Test Tube 9 is held by Hand 3. The area of the Test Tube 9 held by Hand 3 is below the Holder Shield 25. Needle 5 is inserted into Test Tube 9 through Stopper 11. In this way, a liquid such as blood is transferred into the Test Tube 9. However, if Needle 5 misses Stopper 11 or slips off it, Needle 5 will strike the Receptacle Holding Area 29 or Needle Protecting Area 33, as shown in 5″ with dotted lines. Thus, Hand 3 which is holding Test Tube 9 may be protected from the Needle 5. Moreover, because of Ridge 31, which provides a barrier around the perimeter of Needle Protecting Area 33, even if Needle 5 slides across the surface of Needle Protecting Area 33, it will not go off the edge and possibly strike Hand 3.

Test Tube 9 is removed from Holder Shield 25 in the manner of Direction C shown in Fig. 7. This completes the transfer of liquid into Test Tube 9.

Test Tube 9 is removed from Holder Shield 25 according to Direction C as shown in Fig. 7 in order to prevent any possible contamination due to the contact of Stopper 11 with Holder Shield 25. Contact could occur if removal were done in a direction opposite to Direction C. Since removal of Test Tube 9 according to Direction C avoids contamination of the Holder Shield 25, the Holder Shield can be used repeatedly without having to be sterilized or discarded.

Fig. 9 shows another embodiment of the Holder Shield 35. The top view is shown in (a), and the cross-sectional view is shown in (b). The Holder Shield 35 is comprised of Needle Protecting Area 37, in the center of which is a Holding Aperture 39. There is a Ridge 41, which provides a raised area around the perimeter of Needle Protecting Area 37. This Ridge 41 extends both above and below the perimeter of the Needle Protecting Area 37.

The Holder Shield 35 is made of a single piece of plastic which is pliable yet sufficiently strong so as to prevent needle penetration. The surface is rough so as to prevent the needle from slipping or sliding.

The Test Tube 9 is pushed through the Holding Aperture 39 of the Holder Shield 35 until it reaches the Upper Tube Section 91. This situation is shown in Fig. 10. Because of the pliability of the material of Holding Shield 35, the Holding Aperture 39 is enlarged a little so as to allow Test Tube 9 to pass through and to be tightly secured.

By making the two surfaces of the embodiment of Holder Shield 25 identical and by making the two surfaces of the embodiment of Holder Shield 35 identical, either surface can be used. Thus, a user does not need to make a judgement concerning which surface to use.

Although only two embodiments of the Holder Shield (25 and 35) have been described above, other embodiments are possible:

(1) The shape of the Holder Shield need not be circular but could be a square or polygon.

(2) The Holder Shield need not be flat but could be curved.

(3) Receptacles other than test tubes, e.g., flasks and bottles, could be used with the Holder Shield. Also, liquids other than blood could be contained.

## Claims

1. A Needle Shield, which has the characteristic of being an extension of an ordinary needle cap or cover.

2. The form of the extension in said claim 1 is that of a funnel-like shield.

3. A Holder Shield, which has the characteristic of being in the form of a disc which has a hole in the middle which secures receptacles such as test tubes.

4. A Holder Shield of said claim 3, which has the characteristic of having (1) a pliable but needle impervious middle section (Receptacle Holding Area) with a hole in the center (Holding Aperture), and (b) a protection section (Needle Protecting Area) which extends outward from the middle section and is also needle impervious.

5. A holder Shield of said claim 3, which has the characteristic of being made of one piece of needle impervious material.

6. A Holder Shield of said claim 3, which has the characteristic of having a ridge around the outer perimeter of the disc. Such a ridge occurs on both sides of the disc.

FIG.1

PRIOR ART

FIG.2.

PRIOR ART

EP 0 314 278 A1

(a)

(b)

(C)

F I G . 3

FIG. 4

FIG. 5

5 (a)

FIG. 6

5 (b)

FIG. 7

FIG. 9

(a)

(b)

FIG. 8

FIG.10

## DOCUMENTS CONSIDERED TO BE RELEVANT

EP 88307915.4

| Category | Citation of document with indication, where appropriate, of relevant passages | Relevant to claim | CLASSIFICATION OF THE APPLICATION (Int. Cl.4) |
|---|---|---|---|
| X | US - A - 4 610 667 (J.PEDICANO et al.) <br> * Fig. 1-3; abstract * | 1,2 | A 61 M 5/32 |
| X | WO - A1 - 85/03 006 (W.SUMNER) <br> * Fig. 4-6; page 7, line 13 - page 8, line 16 * | 1,2 | |
| X | EP - A1 - 0 160 849 (T.MAYER) <br> * Fig. 1,2; page 5, last paragraph - page 7, paragraph 1 * | 1,2 | |
| X <br> A | US - A - 4 559 042 (T.VOTEL) <br> * Fig. 2; abstract; column 2, line 66 - column 3, line 2 * | 3,5 <br> 4 | |
| X | US - A - 4 573 975 (B.FRIST et al.) <br> * Fig. 1,2; column 2, lines 11-40 * | 3,5 | |

TECHNICAL FIELDS SEARCHED (Int. Cl.4)

A 61 M 5/00

The present search report has been drawn up for all claims

| Place of search | Date of completion of the search | Examiner |
|---|---|---|
| VIENNA | 23-02-1989 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82